# EUROPEAN PATENT APPLICATION

(11) **EP 4 454 638 A1**
(43) Date of publication of application: **30.10.2024**
(21) Application number: 22912054.8
(22) Date of filing: 23.12.2022
(51) Int. Cl.: A61K 9/08, A61K 47/14, A61K 47/10, A61K 31/454, A61P 31/10

(54) **COMPOSITION COMPRISING EFINACONAZOLE FOR TREATMENT OF ONYCHOMYCOSIS**

(30) Priority: 23.12.2021 KR 20210186651
(71) Applicant: Sinsin Pharm Co., Ltd., Sejong 30002 (KR)
(72) Inventor: KIM, Joo-Hyung, Seoul 07793 (KR); HAN, Moon-Seok, Seoul 07793 (KR); LEE, Woo-Young, Seoul 07793 (KR); KIM, Sang-Lin, Seoul 07793 (KR); LEE, Byoung-Ki, Seoul 07793 (KR); KIM, Han-Ki, Seoul 07793 (KR)
(74) Representative: Louis Pöhlau Lohrentz
(86) International application number: PCT/KR2022/021257
(87) International publication number: WO 2023/121421

(57) **Abstract**

Disclosed is a composition comprising efinaconazole for treatment of onychomycosis.

## Description

### TECHNICAL FIELD

The present invention relates to a composition for treating onychomycosis containing efinaconazole.

### BACKGROUND ART

Treatments for onychomycosis are mainly developed in the form of a liquid preparation including efinaconazole, tavaborole, ciclopirox, amorolfine, terbinafine or salts thereof as a main ingredient. These liquid preparations are in the form of solutions in which nail penetration enhancers, chelating agents, antioxidants, non-volatile solvents, wetting agents, antioxidants, pH regulators, and the like are dissolved in water and/or ethanol to increase the stability of the active ingredient and the drug absorption rate into the nails.

A medicine containing efinaconazole for the treatment of onychomycosis is currently on the market under the tradename Jublia^{®} (Kaken Pharmaceutical, Co., Ltd. Japan; containing 10% efinaconazole). This medicine is a non-film-forming liquid-type formulation that uses alkyl lactate as a nail absorption enhancer, and the alkyl lactate reacts with citric anhydride, which is a pH regulator, to form triglycerides, thereby further promoting the nail absorption and penetration effects.

Many other studies have been conducted on the treatment of onychomycosis, but no medicine other than Jublia has been developed yet.

### DETAILED DESCRIPTION OF THE INVENTION

### TECHNICAL PROBLEM

The present invention provides an onychomycosis treatment composition with improved nail penetration rate of efinaconazole.

### TECHNICAL SOLUTION

The present invention relates to a pharmaceutical composition including: efinaconazole or a pharmaceutically acceptable salt thereof; and a lipid-soluble nail absorption enhancer. The composition of the present invention may further include a non-volatile solvent. The composition of the present invention may be a liquid formulation for treatment of onychomycosis.

The composition of the present invention may further include one or more selected from the group consisting of water and ethanol as solvent. Preferably, volatile ethanol is used as a solvent, and water may optionally be included in a small amount (e.g., less than 5% by weight based on the total weight of the composition).

In the composition of the present invention, the lipid-soluble nail absorption enhancer is preferably a propylene glycol fatty acid ester. In the present invention, propylene glycol monocaprylate, propylene glycol dicaprylate/dicaprate, propylene glycol monolaurate, propylene glycol monooleate, propylene glycol monostearate, or the like may be preferably used as the propylene glycol fatty acid ester. Most preferably, propylene glycol monocaprylate is used.

In the present invention, the lipid-soluble nail absorption enhancer may be included in an amount of 1% to 20% by weight, preferably 5% to 15% by weight, based on the total weight of the composition.

In the present invention, a preferably non-volatile solvent is diisopropyl adipate, which may be included in an amount of 1% to 20% by weight, preferably 5% to 15% by weight, based on the total weight of the composition.

In addition, the composition of the present invention may further include one or more among a chelating agent, a wetting agent, a pH regulator, and an antioxidant.

Chelating agents that may be used in the present invention include any commonly used chelating agents, but diethylenetriaminepentaacetic acid is preferably used. The chelating agent may be included in an amount of less than 2% by weight based on the total weight of the composition of the present invention.

Wetting agents that may be used in the present invention include any commonly used wetting agents, but cyclomethicone is preferably used. The wetting agent may be included in an amount of 5% to 15% by weight based on the total weight of the composition of the present invention.

### ADVANTAGEOUS EFFECTS

The pharmaceutical composition according to the present invention has excellent percutaneous penetration rate and also excellent nail absorption rate.

### DESCRIPTION OF THE DRAWINGS

FIG. 1 shows a graph illustrating the dermal penetration amount per unit area in each hour for 24 hours when the composition according to the present invention is applied.
FIG. 2 shows a graph illustrating the penetration rate for 1 day when the composition according to the present invention is applied.

### EXAMPLES

Hereinafter, the present invention will be described in detail based on examples in order to help understanding the present invention. However, the following examples are only intended to illustrate the content of the present invention, and the spirit or scope of the present invention is not limited by the following examples in any way. The examples of the present invention are provided to more completely explain the present invention to those having average knowledge in the art.

### Examples 1 and 2: Preparation of composition

The compositions of Examples 1 and 2 and Comparative Examples 2 and 3 were prepared according to the ingredients and contents shown in Table 1 below.

**[Table 1]**

| Ingredient | | Example 1 | Example 2 | Comparative Example 2 | Comparative Example 3 |
|---|---|---|---|---|---|
| Active ingredient | efinaconazole | 10.0% | 10.0% | 10.0% | 10.0% |
| Solvent | ethanol | 53.8% | 65.8% | 53.8% | 53.8% |
| Nail absorption | propylene glycol monocaprylate | 10.0% | 10.0% | - | - |
| enhancer | Tri-block copolymer | - | - | 10.0% | - |
| | polyoxyethylene (23) laurylate | - | - | - | 10.0% |
| Non-volatile solvent | diisopropyl adipate | 12.0% | - | 12.0% | 12.0% |
| Wetting agent | cyclomethicone | 13.0% | 13.0% | 13.0% | 13.0% |
| Chelating agent | Diethylenetriamine pentaacetic acid | 0.00025% | 0.00025% | 0.00025% | 0.00025% |
| Antioxidant | Butylated hydroxytoluene | 0.1% | 0.1% | 0.1% | 0.1% |
| pH regulator | Citric acid anhydrous | 0.1% | 0.1% | 0.1% | 0.1% |
| Purified water | | 0.99975% | 0.99975% | 0.99975% | 0.99975% |
| Total | | 100.0% | 100.0% | 100.0% | 100.0% |

### Experimental Example 1: Evaluation of artificial dermal penetration rate

The dermal penetration rate of the compositions of Examples 1 and 2, Comparative Examples 2 and 3, and the efinaconazole-containing topical medicine for treatment of onychomycosis currently available on the market (Comparative Example 1) was evaluated by the method described below.

Strat-M^{™} Membrane (transdermal diffusion test membrane, Millipore) was used as an artificial skin, and 200 µL of each of the compositions of Examples 1 and 2 and Comparative Examples 1 to 3 was applied to each donor compartment. The dermal penetration rate test was performed in triplicate each (n=3), and the dermal penetration rate test conditions using the Franz diffusion cell are described below.
- Diffusion area: 1.767 cm²
- Sample loading: 200 µL each of the compositions of Examples 1, 2, and Comparative Examples 1 to 3
- Receiver compartment: 7 mL of phosphate buffered saline (PBS) (pH 7.4) containing 1.5 % (w/v) Brij^{™} L23 (Sigma-Aldrich) and 0.1 % (w/v) sodium azide
- Stirring speed: 600 rpm
- Temperature: 32 °C
- Sample collection: 200 µL each collected from each receiver compartment at 2 hours, 4 hours, 8 hours, 12 hours, and 24 hours, and then supplemented with 200 µL each with the PBS containing 1.5 % (w/v) Brij^{™} L23 (Sigma-Aldrich) and 0.1 % (w/v) sodium azide.

The amount of efinaconazole present in the collected samples was analyzed by high-performance liquid chromatography (HPLC). The HPLC analysis was performed under the conditions described below.

### <HPLC Conditions>

- Column: Inert Sustain C18 (250 × 4.6 mm, 5 µm)
- Column temperature: 50 °C
- Mobile phase: (Buffer solution with concentrations of sodium 1-octanesulfonate and potassium dihydrogen phosphate of 3.0 g/L and 6.8 g/L, respectively, and pH 3.7) : (acetonitrile: methanol = 60:40) = (55:45, v/v)
- Flow rate: 1.8 mL/min
- Detection: 210 nm
- Sample injection amount: 20 µL

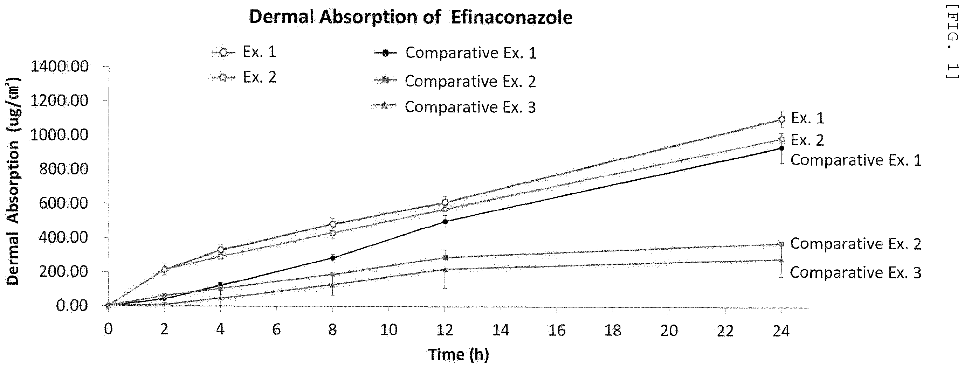

The analytical results are shown in Table 2 and FIG. 1 below.

**[Table 2]**

| Preparation | 12 hr | 24 hr |
|---|---|---|
| | Amount of penetration per unit area (µg/cm²) | Amount of penetration per unit area (µg/cm²) |
| Example 1 | 610.77 | 1103.21 |
| Example 2 | 570.77 | 987.12 |
| Comparative Example 1 | 499.24 | 884.58 |
| Comparative Example 2 | 289.08 | 373.99 |
| Comparative Example 3 | 219.12 | 282.24 |

As can be seen from the results shown in Table 2, the compositions of Examples 1 and 2 according to the present invention had higher dermal penetration concentrations than those of Comparative Examples 1 to 3. In particular, Example 2 exhibited a higher penetration concentration than that of the comparative examples with only the nail absorption enhancer without a non-volatile solvent.

The composition of Example 1, prepared by further adding a non-volatile solvent, also significantly increased the dermal penetration concentration compared to the comparative examples. Therefore, it is more preferable to use a nail absorption enhancer together with a non-volatile solvent.

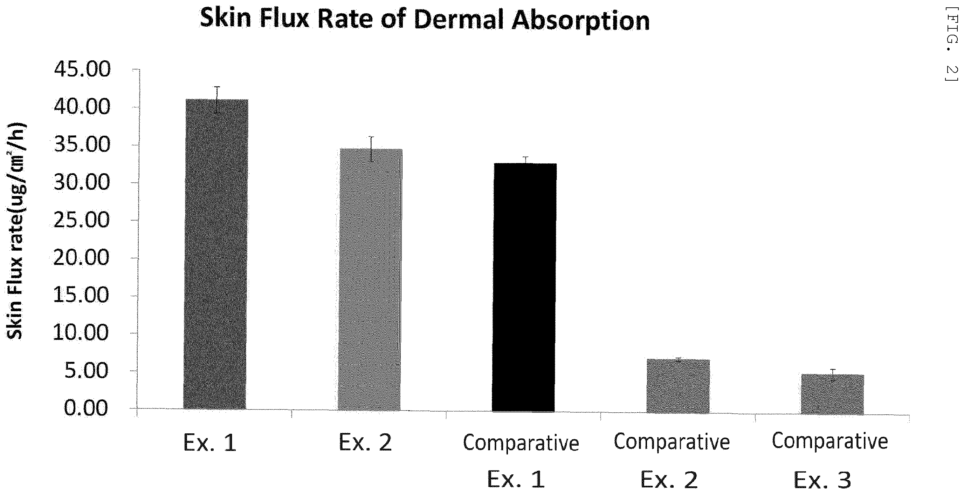

From the above results, the average penetration rate for the last 12 hours of one day for each sample (i.e., [dermal penetration amount per unit area at 24 hours - penetration amount per unit area at 12 hours]/12 hours) was calculated and plotted in FIG. 2.

As can be seen from the results of FIGS. 1 and 2, both Examples 1 and 2 exhibited significantly increased penetration amount per unit area and penetration rate compared to the comparative examples.

Therefore, the topical solution containing efinaconazole according to the present invention exhibits an improved percutaneous penetration rate of efinaconazole.

### Experimental Example 2: Evaluation of amount of main ingredient absorption into nails

To evaluate the amount of main ingredient absorption into the nails, the compositions of Example 1 and Comparative Example 3 were applied on the surface of cloven hoofs obtained from pigs, and the amount was analyzed after 24 hours. Although cloven hoofs of pigs are thicker than the human nails, they are often used for alternative experiments.

Cloven hoofs of pigs obtained in small quantities from a slaughterhouse were kept frozen until used in the tests.

The day before the test, the cloven hoofs were washed with clean water, cut into pieces with a diameter of 15 cm and a thickness of about 3 to 5 mm to prepare coin-shaped cloven hoof plates, and soaked in saline solution for 24 hours to hydrate. On the day of the test, moisture from the hydrated cloven hoof plates was removed, and the composition of Example 1 or Comparative Example 3 was evenly applied to the surface of the cloven hoof plates, and the initial application amount was calculated.

The cloven hoof plate to which the composition was applied was placed in a petri dish, covered with plastic wrap, and allowed to stand in an incubator chamber at 32 °C for 24 hours. Thereafter, each surface of the cloven hoof plates to which the composition was applied was wiped about 10 times with a cotton swab soaked in methanol.

Thereafter, the cloven hoof plates were cut into small pieces, placed in 7 mL of the extraction solution [1 N sodium hydroxide solution:methanol = 1: 1 (v/v)], and extracted at 60 °C for four hours.

Finally, the extracted solution was placed in a centrifuge and centrifuged by revolving at 4,000 rpm for 10 minutes, and only the supernatant was collected and the amount was analyzed under the same HPLC conditions as Experimental Example 1 to evaluate the absorption rate into the cloven hoofs.

The analytical results are shown in Table 3 below.

**[Table 3]**

| Classificat ion | Amount of application (mg) | Amount of absorption to the cloven hoofs (mg) | Absorption rate (%) | Average absorption rate (%) |
|---|---|---|---|---|
| Example 1 (Sample 1) | 13.27 | 1.020 | 7.68 | 6.46 |
| Example 1 (Sample 2) | 13.08 | 0.966 | 7.38 | 6.46 |
| Example 1 (Sample 3) | 1.92 | 0.101 | 5.28 | 6.46 |
| Example 1 (Sample 4) | 2.85 | 0.157 | 5.50 | 6.46 |
| Comparative Example 3 (Sample 1) | 12.56 | 0.329 | 2.62 | 2.40 |
| Comparative Example 3 (Sample 2) | 13.24 | 0.339 | 2.56 | 2.40 |
| Comparative Example 3 (Sample 3) | 4.10 | 0.089 | 2.16 | 2.40 |
| Comparative Example 3 (Sample 4) | 4.24 | 0.096 | 2.26 | 2.40 |

As can be seen from the results shown in Table 2, the composition of Example 1 according to the present invention exhibited a significantly higher average absorption rate into the cloven hoofs than that of Comparative Example 3. Therefore, the efinaconazole-containing topical solution according to the present invention exhibits an improved absorption rate into the nails.

### INDUSTRIAL APPLICABILITY

The efinaconazole-containing composition according to the present invention has excellent skin penetration rate and thus can be effectively used as a treatment for onychomycosis.

## Claims

1. A pharmaceutical composition comprising: efinaconazole or a pharmaceutically acceptable salt thereof; and a lipid-soluble nail absorption enhancer.

2. The pharmaceutical composition according to claim 1, further comprising a non-volatile solvent.

3. The pharmaceutical composition according to claim 1 or 2, further comprising one or more selected from the group consisting of water and ethanol.

4. The pharmaceutical composition according to claim 1 or 2, wherein the lipid-soluble nail absorption enhancer is a propylene glycol fatty acid ester.

5. The pharmaceutical composition according to claim 4, wherein the propylene glycol fatty acid ester is selected from the group consisting of propylene glycol monocaprylate, propylene glycol dicaprylate/dicaprate, propylene glycol monolaurate, propylene glycol monooleate, and propylene glycol monostearate.

6. The pharmaceutical composition according to claim 2, wherein the non-volatile solvent is diisopropyl adipate.

7. The pharmaceutical composition according to claim 1 or 2, further comprising one or more among a chelating agent, a wetting agent, a pH regulator, and an antioxidant.

8. The pharmaceutical composition according to claim 7, wherein the pharmaceutical composition is a liquid formulation for treatment of onychomycosis.
